# EUROPEAN PATENT APPLICATION

(11) **EP 4 550 345 A1**
(43) Date of publication of application: **07.05.2025**
(21) Application number: 24155293.4
(22) Date of filing: 01.02.2024
(51) Int. Cl.: G16H 20/30, G06F 3/01, G16H 30/20, G16H 40/60

(54) **A METHOD FOR MONITORING AND VISUALIZING THE PROCESS OF SPINE RELIEVING REHABILITATION AND A SYSTEM FOR IMPLEMENTING THE METHOD**

(30) Priority: 03.11.2023 PL 44663423
(71) Applicant: Akademia Gorniczo-Hutnicza im. Stanislawa Staszica w Krakowie, 30-059 Krakow (PL)
(72) Inventor: Kwasniewski, Jerzy, 30-211 Kraków (PL); Molski, Szymon, 31-901 Kraków (PL); Nawrocki, Marcin, 32-020 Wieliczka (PL)
(74) Representative: Wlasienko, Jozef

(57) **Abstract**

A method for monitoring and visualizing the process of spine relieving rehabilitation uses virtual and mixed reality technologies as well as a virtual 3D model of the spine of a patient undergoing rehabilitation in this way. The method involves real-time monitoring a mixed reality image composed of a hologram of the patient with a superimposed virtual image of the spine model adapted to a specific patient and an image of changes in its position that are generated by the rehabilitation process. The virtual 3D spine model is adapted to the patient's anthropometric parameters and to the pathological condition of his/her spine, and then suspension points are determined for sling hangers of a rehabilitation device, in which virtual "cross-sectional planes" are installed, and based on such an adapted 3D virtual spine model a rehabilitation program is designed. During the monitoring of the spine relieving rehabilitation process, the correctness of the designed rehabilitation program is verified and possible modifications are introduced.

A system for implementing the method is based on the use of a device for spine relieving rehabilitation, protected by AGH patent no. PL 226008, equipped with a set of moving cameras scanning the patient undergoing rehabilitation and with sensors for the direction and amount of movement of each actuating element implementing the rehabilitation program. The implementation of the method and the operation of the system are controlled by computer-assisted systems with appropriate software.

## Description

The subject of the invention is a method for monitoring and visualizing the process of spine relieving rehabilitation. The subject of the invention is also a device enabling the implementation of the method for monitoring and visualizing the process of spine relieving rehabilitation.

Spine diseases are one of the most common ailments that make everyday functioning much more difficult. Spine diseases are a very extensive group of diseases. These include, among others: congenital anomalies, posture defects, degenerative changes, discopathy, inflammation and cancer. The treatment of spine diseases usually involves physiotherapy, i.e. motor rehabilitation, and in severe cases, surgery.

In case of severe symptoms or repeated episodes of pain, spine rehabilitation is used, which is the basic support for treatment, and often its replacement, and its long-term goal is to strengthen the muscular corset supporting the spine.

Medical engineering seeks for methods and ways to improve the process of patient rehabilitation and strives to facilitate the work of physiotherapists dealing with spine rehabilitation and to improve the quality of their education by creating new devices, apparatus and systems used in this field.

One of the most effective methods of spine rehabilitation is the method of suspending the patient, which allows for exercises to be performed without any load. Relieving the load in spine rehabilitation is very important because it allows to perform exercises that increase the range of motion in the joints in a safe and effective way.

The type, scope and parameters of rehabilitation exercises are determined based on clinical diagnostics, supported by imaging diagnostics in the form of X-ray, CT (computed tomography) and MRI (magnetic resonance imaging). Virtual 3D models of patients' spines, developed on the basis of imaging diagnostics, are helpful in diagnosing and determining a rehabilitation program and they allow for the analysis of spine problems and the definition of therapeutic actions.

The search for new methods of diagnosis and rehabilitation leads to the use of virtual reality technology. Virtual therapy is interactive in real time and allows for quantitative and qualitative assessment of the ranges of mobility of selected spine sections while performing movement exercises in three dimensions.

In 2013, a technical solution was developed entitled: "A device for muscle stimulation and spine rehabilitation", patented under no. PL 226008, that relates to a rehabilitation device, and a solution extending the previous solution with the possibility of implementing lateral movement entitled "A device for spine rehabilitation and a method of spine rehabilitation using a device for spine rehabilitation", application no. P.431381 and US patent no. US 11484458 B2 entitled DEVICE FOR SPINE REHABILITATION AND METHOD OF SPINE REHABILITATION USING SAID DEVICE FOR SPINE REHABILITATION has been granted.

The solution according to the invention, protected by patents Nos. PL 226008 and US 11484458 includes a support frame, at least four sling hangers supporting the spine in a substantially horizontal position, the sling hangers being attached to the support frame with the possibility of sliding in the longitudinal and transverse directions to the axis of the spine and with the possibility of moving in the vertical direction, and also tilting relative to the long axis of the spine.

The essence of the invention is that each sling hanger is connected at the ends to two linear actuators, which are suspended in pairs to a horizontal supporting frame in a position perpendicular to the spine axis, and moreover, the movement of each linear actuator is controlled individually by a computer-assisted control system with medical software.

The linear actuators connected in pairs to each sling hanger are connected to the supporting frame through sliding units in the longitudinal and transverse directions to the spine axis.

In the application no. CN 115471628 (A) - 2022-12-13 entitled: Spine mechanics display method and system based on augmented reality and three-dimensional finite, an invention is disclosed that provides a method and system for displaying spine mechanics based on augmented reality and three-dimensional finite elements, and the method includes the steps of: carrying out the segmentation and reconstruction of a three-dimensional cone model according to obtained medical image data; carrying out materialization on the three-dimensional cone model after smooth pretreatment, and carrying out grid division after adding intervertebral discs and assembling screws to obtain a three-dimensional grid model; performing finite element analysis on the three-dimensional volume mesh model to obtain a finite element model, solving stress of each point of the spine segment according to the finite element model and node loads obtained in real time, and outputting and displaying the stress; wherein when a new geometric model is received, independent cutting and mesh generation are performed on the geometric model, the intersection surface of the geometric model and the finite element model is segmented, and a part intruding into the finite element model is removed; different grids are connected, a finite element model is re-analyzed, and stress solving result is updated, so that a seamless interface is established in a physical environment and the interaction efficiency between a user and the model is improved.

The solution titled: Augmented reality assisted spine internal fixation screw implantation method and system No. CN 113476140 (A) - 2021-10-08 presents an invention that discloses augmented reality assisted spine internal fixation screw implantation method and system, and relates to the technical field of spinal pedicle screw implantation. The method comprises the following steps: creating a virtual spine three-dimensional model according to patient lesion image data; making an operation plan according to the virtual spine three-dimensional model, and obtaining a virtual spine three-dimensional model with the operation plan; importing the virtual spine three-dimensional model with the operation plan into VR equipment to obtain a retina projection 3D model with the operation plan; a doctor wearing AR equipment, performing matching calibration on the retina projection 3D model with the operation plan and the real spine model, performing locking after calibration is completed, obtaining the calibrated projection 3D model with the operation plan, and completing an operation on the real spine model according to the operation plan shown by the projection 3D model; and evaluating the accuracy of nail planting. The nail implanting accuracy is remarkably improved, and the risk of navigation drifting in an operation is greatly reduced.

The solution titled: AUGMENTED REALITY SYSTEM FOR PHANTOM LIMB PAIN REHABILITATION FOR AMPUTEES US2023058936 (A1) 2023-02-23 presents an invention relating to a system for neuromuscular rehabilitation of a patient having an affected limb comprising: a feedback member arranged to give real-time visual feedback; a plurality of electrodes arranged to acquire an electric signal corresponding to an intent to move said affected limb; a control unit configured to: perform pattern recognition of said electric signals, wherein at least one feature in said electric signal is used to predict motion intent of said affected limb adjacent to at least one joint, such aggregated motions of said affected limb are predicted; based on output signals from said performed pattern recognition, control said feedback member to perform actions corresponding to said motions, whereby said actions of said feedback member are individually and simultaneously controlled by said patient via said intended motions.

The application titled: AUGMENTED REALITY TOOL FOR IMPLANT SURGERY AND IMPLANT SURGERY INFORMATION VISUALIZATION METHOD - WO2021112312 (A1) - 2021-0610 presents an invention relating to an augmented reality tool for implant surgery and an implant surgery information visualization method, and, particularly, to an augmented reality tool for implant surgery and an implant surgery information visualization method, which virtualize and display, on an actual camera image, an implant guide model for the position at which an implant will be implanted and a driver that is to implant the implant during implant surgery such as spine surgery, and which visualize surgery information including information about the relationship between the implant guide model and the displayed driver, so as to display same on the camera image in real time, thereby providing convenience and accuracy in implant surgery.

The rehabilitation process for patients with spine problems involves the patient performing a series of movements forcing the spine into the desired position and exercising specific muscle groups. When planning the rehabilitation process based on clinical and imaging diagnosis, decisions are made about the scope, frequency, number of repetitions and changes in forcing movement cycles, based on assumptions resulting from the practice and experience of the person planning the rehabilitation. The correct development of an appropriate rehabilitation plan is particularly important in the case of the relieving rehabilitation process, in which the patient's forced movements are caused by mechanical forces independent of the patient. In the above-mentioned state of the art, there are no solutions enabling the observation of the suspended patient movements generated by actuators and the simultaneous observation of his spine movements. Such a solution would enable monitoring of the rehabilitation process, while improving its effectiveness and safety.

The process of spine relieving rehabilitation is carried out based on the diagnosis developed through the analysis of materials obtained from diagnostic imaging in the form of X-ray, CT and MRI. A series of patient movements is established that force the desired spine positions and specific muscle group exercise. On a device for spine relieving rehabilitation, only the patient's movements are observed, without observing the movement of his spine. It would be advisable to simultaneously observe the actual displacements of the spine while forcing the patient into a "suspended" position, taking into account the medical limitations of movement of individual vertebrae and the entire spine.

The inventive problem is a method for monitoring and visualizing the process of spine relieving rehabilitation, enabling analysis, in real time, of the effects of muscle stimulation or spinal rehabilitation treatments with a dynamic impact optimally matched to the existing disease, with freely diversified, medically developed kinematics performed on a real device for spine relieving rehabilitation, with simultaneous visualization of the movement of a virtual spine model, as well as a system for implementing this method. At the same time, the inventive problem is a method of designing and modifying a relieving rehabilitation program based on the virtual spine model.

The essence of a method for monitoring and visualizing the process of spine relieving rehabilitation, according to the invention, using a device for spine relieving rehabilitation and virtual and mixed reality technologies, as well as a virtual 3D model of the spine of a patient undergoing rehabilitation in this way is characterized by real-time monitoring a mixed virtual reality image composed of a real image of the patient, a hologram of the patient and a virtual image of his/her spine model superimposed on it, as well as changes in the position thereof, generated by the rehabilitation process. The possibility of implementing such monitoring requires a number of actions. First of all, a 3D virtual spine model corresponding to the anthropometric data of the patient for whom the relieving rehabilitation process is being prepared, is downloaded from a database of typical virtual spines, containing at least 6 different models. Then, a selected typical 3D spine model is modified based on imaging diagnostics and the patient's anthropometric parameters, and natural curvatures and all pathological changes shown in the imaging documentation are introduced. The 3D spine model adapted in this manner to a specific patient and his/her medical condition is scaled based on the patient's anthropometric parameters and his/her holographic avatar. The personalized 3D spine model is archived in a database of virtual images of patients' spines. The adapted, virtual 3D spine model of the patient, for which the relieving rehabilitation process is being prepared, is downloaded from the database of virtual images of patients' spines and, based on his/her anthropometric dimensions, at least four and preferably five suspension points are determined, which determine the axes of sling hangers. After determining the suspension points, virtual cross-sectional planes related to the spine model, being perpendicular to the long axis of the virtual spine model, are generated at the suspension points in the adapted virtual 3D spine model of the patient. For the rehabilitation process with five suspension points, five cross-sectional planes are determined, namely a cross-sectional plane at the head suspension point, a cross-sectional plane at the shoulder suspension point, a cross-sectional plane at the hip suspension point, a cross-sectional plane at the knee suspension point, and a cross-sectional plane at the feet suspension point. In the virtual spine model of the patient, based on his/her anthropometric dimensions, distances of individual planes from a base plane, which is the hip plane, are determined. At a distance x1 from the hip plane the shoulder plane is established. At a distance of x2 from the hip plane the head plane is established. At a distance of x3 from the hip plane the feet plane is established. And at a distance of x4 from the hip plane the knee plane is established. The relieving rehabilitation process consists of individual stages. At each stage of the relieving rehabilitation process, a series of movements of sling hangers is performed to force the desired spine position and, additionally, exercises of specific muscle groups of the patient. A relieving rehabilitation program is determined for individual stages of rehabilitation, based on the scaled and adapted virtual spine model of the patient. On a computer touch screen or using holographic glasses, individual virtual cross-sectional planes are moved vertically and/or are rotated around the suspension point and/or are tilted from the vertical, thereby setting the virtual spine model in the desired rehabilitation position for the final stage of the rehabilitation process. Data regarding the displacements of the cross-sectional planes in relation to the initial position and the decreed time for maintaining the forced position are transferred to a programming block of the rehabilitation process. For each subsequent stage of the rehabilitation process, the same procedure is followed to design the required spine position. In the programming block of the rehabilitation process, information about the change in the position of individual planes is transformed into signals, by means of which the operation of individual actuating elements is controlled, causing the movement of virtual cross-sectional planes, thereby creating a sequence of signals controlling the actuating elements for implementing the rehabilitation position for each subsequent stage of the rehabilitation process. Signal sequences for each stage are stored in a block constituting a database of rehabilitation programs. After designing all stages of the rehabilitation program dedicated to the patient, it is downloaded and the actuating elements, preferably linear actuators, are positioned, each of which in pairs or individually is intended for one sling hanger. The sling hangers are set in such a way that their axes run through the patient's suspension points, and the planes of the patient's suspension points perpendicular to the long axis of the spine, constituting the virtual cross-sectional planes, coincide with the strings of the actuating elements. The patient is placed on the sling hangers arranged at set distances x1 to x4. Then, the patient is relieved to the starting position and a holographic image of the patient is generated in a visualization block using an image signal from scanning cameras that move at a constant speed over structural elements of the device for spine rehabilitation and archived in the database of patients' virtual images. Then the relieving rehabilitation program begins. The movement of the actuating elements of the device for rehabilitation is carried out based on the program controlling the device for rehabilitation, using the settings of selected movement planes set by a rehabilitation doctor/physiotherapist on the virtual image of the spine.

Signals representing the amount and direction of movement of the actuating elements of the device for rehabilitation that implement subsequent stages of the rehabilitation program are transferred to a spine movement visualization system. Based on the sequence of signals representing the direction and range of the actuating element movements carried out for a given stage of the rehabilitation program, a new image of the spine model of the rehabilitated patient and an image of the position of virtual cross-sectional planes connected to the spine model are generated in the spine movement visualization system. Using the position of five virtual cross-sectional planes connected to the spine model, its movement is visualized in two directions vertical and horizontal and additionally in rotation around the long axis of the spine for planes equipped with pairs of actuating elements, to the extent resulting from the applied stroke or movement of each actuator from the pair of actuating elements related to a specific sling hanger. In a mixed reality system, in the holographic glasses, the real image of the device for rehabilitation from a first environment from the visualization block is combined with the computer-generated hologram of the patient from a second environment from the database of patient holograms and with the virtual image, correlated with the hologram, of the model of his/her spine from a third environment from the spine model database. The course of the rehabilitation procedure is monitored in real time by observing correlated images, the real image from the first environment and the patient's hologram generated in the visualization block, as well as the virtual image of the model of the rehabilitated patient's spine with marked cross-sectional planes, which is superimposed on the semi-transparent hologram of the patient. Based on the image of the patient's actual movement on the device for rehabilitation and the image of the generated movement of the spine model, in the holographic glasses to which the virtual image generated in the visualization block is transferred, the correctness of the range of movements set in the program controlling the device for rehabilitation is verified by computer and the necessary changes or restrictions for the settings specified in the unverified program.

The initial relieving of the patient, constituting the starting position for the rehabilitation process, is carried out by moving the strings of all sling hangers in the direction perpendicular to the plane of a bed by the same amount allowing to obtain space for executing all stages of the rehabilitation program or by lowering the bed by an amount allowing to obtain the required space and the program is completed is by setting a position analogous to the starting position and lowering the patient onto the bed or raising the bed to a position in which the patient's relieving position is eliminated.

The correlated image of the course of a rehabilitation session obtained during the implementation of the method for monitoring and visualizing the process of spine relieving rehabilitation, which image consists of the real image from the first environment, the patient hologram from the second environment and the image of the virtual spine model from the third environment, is stored in a cloud or on a server constituting the user's library.

The range of motion of the actuating elements of the device for rehabilitation, correlated with the movement of the virtual spine model, visualized on the virtual spine model, in the third environment of the patients' spine model base, is limited by the permissible range of motion of the vertebrae and spine, used by medical specialists in clinical trials.

The essence of the system for monitoring and visualizing the process of spine relieving rehabilitation, using a device for spine relieving rehabilitation equipped with a set of scanning cameras and virtual and mixed reality technologies, as well as a virtual 3D model of the spine of a patient undergoing rehabilitation in this way, is characterized in that to the longer sides of a rectangular horizontal supporting frame of the device for spine relieving rehabilitation treadmills are permanently mounted, each having one scanning camera slidingly mounted therein.

Inside the horizontal supporting frame of the device for spine relieving rehabilitation five sliding beams mounted, to which actuating elements, preferably linear actuators, are slidably attached, along with strings and sling hangers attached to them, wherein the first beam is shifted relative to the central fixed beam by a distance of x1 set for the examined patient, the second beam by a distance x2, the third beam by a distance x3, and the fourth beam by a distance x4, which are determined on the basis of anthropological parameters of the examined patient.

Each actuating element of the device for rehabilitation is equipped with a sensor determining the amount and direction of the actual movement of a single string of each sling hanger, and each beam on which the actuating elements of the sling hangers are mounted is equipped with a sensor determining the amount of axial movement of the beam, with all movement signals for each element being transmitted to a spine movement visualization system.

The advantage of the method according to the invention is the ability to improve the effectiveness of relieving rehabilitation and to increase the safety of its implementation. The use of a virtual 3D model of the spine adapted to anthropometric dimensions of a patient, equipped with virtual cross-sectional planes arraged at the suspension points and related to the spine model, enables easy and precise planning of the rehabilitation process tailored to a specific patient. The transformation of the displacements of the cross-sectional planes allows for easy creation of a program controlling the device for spine relieving rehabilitation of a specific patient. Real-time observation of a correlated hologram of the patient with the model of his/her spine allows to verifiy the correct operation of the planned rehabilitation program. It also enables ongoing intervention in the implementation of the rehabilitation program and its modification resulting from the actual conditions of the rehabilitation process. In this way, a unique tool is also created for training physiotherapists in the use of the device in rehabilitation by suspending a patient. The method according to the invention also facilitates the assessment of the suitability of the selected device control program for a given disease entity.

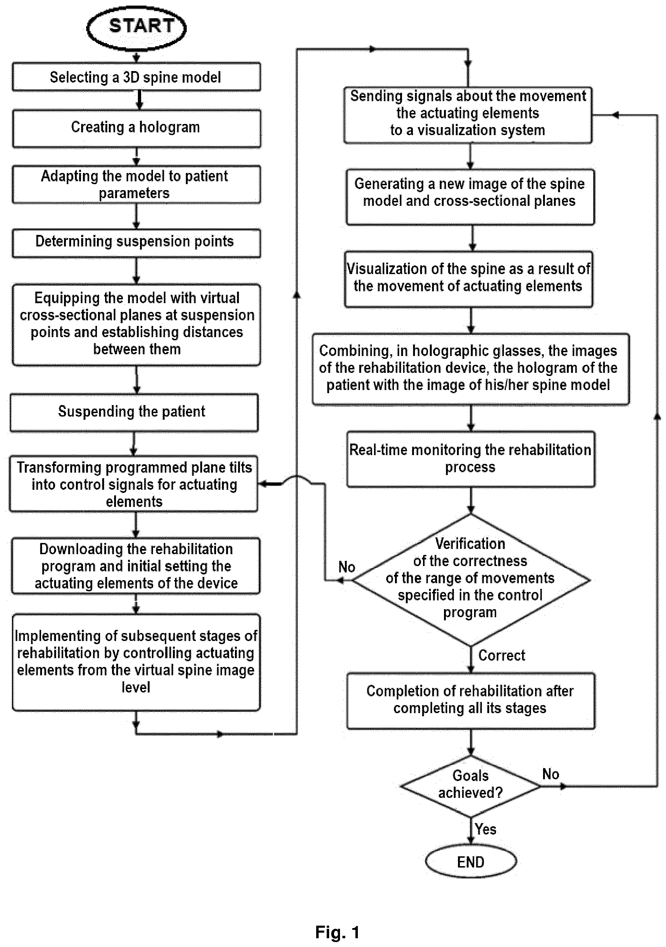

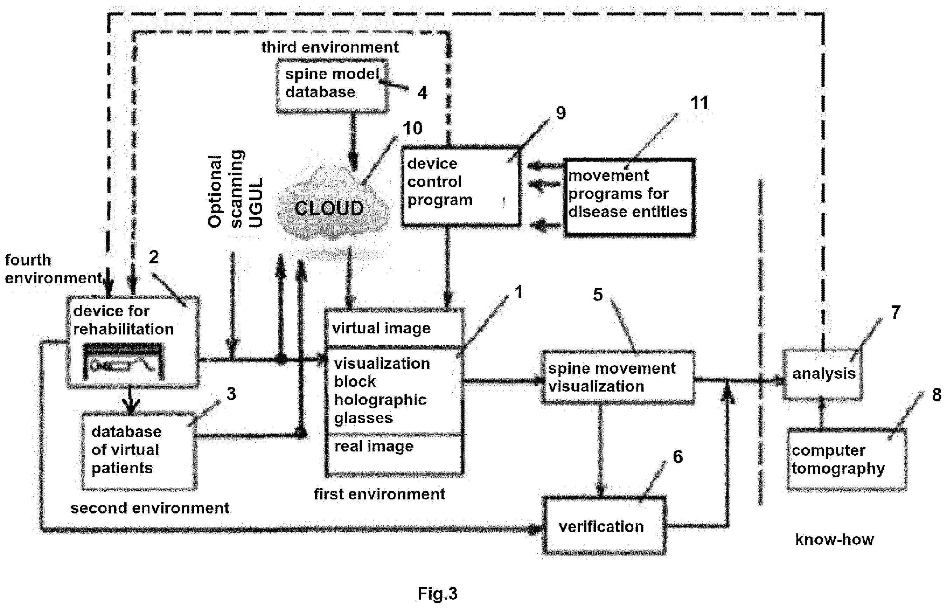

The method and system according to the invention are explained in more detail on the basis of an exemplary embodiment shown in the drawing, in which Fig. 1 shows a block diagram of an algorithm for a rehabilitation method according to the invention, Fig. 2 schematically shows a device for relieving rehabilitation during rehabilitation, and Fig. 3 shows schematically an arrangement of a system for monitoring and visualizing the process of spine relieving rehabilitation.

The implementation of the method for monitoring and visualizing the process of spine relieving rehabilitation is based on the use of a device for relieving rehabilitation, protected by patents nos. PL 226008 and US 2021/0100712 A1 owned by AGH. For the implementation of the method also virtual and mixed reality technologies, as well as a virtual 3D model of the spine of a patient undergoing rehabilitation in this way are used. The method involves real-time monitoring of a mixed reality image consisting of a hologram of the patient with a superimposed virtual image of the patient's spine model and an image of changes in its position, which are generated by the rehabilitation program.

The implementation of the method for monitoring and visualizing the process of spine relieving rehabilitation requires a preparatory stage in which the virtual 3D spine model is adapted to the patient's anthropometric parameters and to the pathological condition of his/her spine, and then suspension points for sling hangers of the device for rehabilitation are determined. Then, virtual "cross-sectional planes" are installed at the suspension points and a rehabilitation program is designed based on the virtual 3D spine model adapted to the parameters and features of the patient's spine.

The method for monitoring and visualizing the process of spine relieving rehabilitation is performed each time for a specific patient. In an embodiment of the invention, the patient was a man aged 60+, height 176 cm, weight 89 kg, hip width 42 cm, shoulder width 52 cm, distance from the 7th cervical vertebra to the center of the ear of 9 cm, and knee distance from the hip bone equal to 54 cm and the distance from the heel to the knee equal to 48 cm. Based on the patient's anthropometric data, a typical model for a man with a height of 170-180 cm was selected from a database of typical virtual 3D spines. Based on the anthropometric parameters and patient imaging diagnostics, the selected virtual 3D spine model was adapted to the features and dimensions of the patient for whom the rehabilitation program is being prepared. The natural curvatures resulting from the image documentation and all pathological changes related to the spine were introduced into the dimensioned virtual spine model. The virtual 3D spine model modified in this way and adapted to the patient and his/her medical condition is marked in a way that identifies the patient and is archived in a database of virtual images of patients' spines 3.

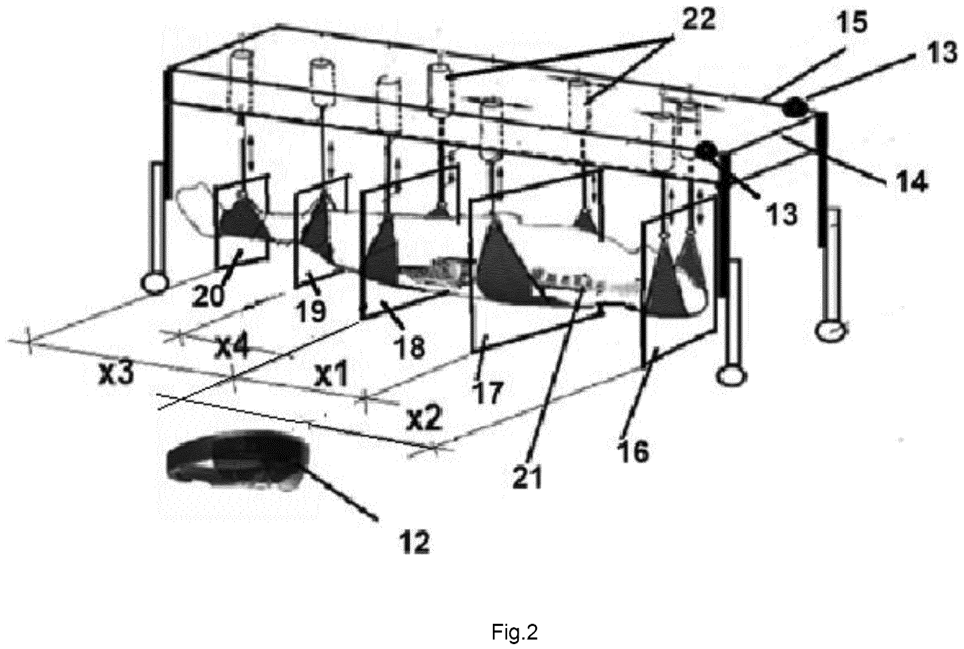

In the next step, the adapted virtual 3D spine model of the patient was downloaded and, based on his/her anthropometric dimensions, five support points were determined that determine the axes of sling hangers. At the support points, virtual cross-sectional planes were generated, perpendicular to the long axis of the spine planes in the adapted virtual 3D spine model of the patient and to a virtual line extending the axis of the virtual spine with such a length that the total length in the line on which the suspension points were designated was 176 cm in total and corresponded to the patient's height. Fig. 2 shows the determined virtual cross-sectional planes connected to the axis of the spine model, wherein two of them: the cross-sectional plane 18 at the hip suspension point, and the cross-sectional plane 17 at the shoulder suspension point, are connected to the spine model, while the plane 16 at the head suspension point is connected to the virtual line extending the spine axis above the first vertebra, and the plane 19 at the knee suspension point and the plane 20 at the feet suspension point are connected to the virtual line extending the spine axis below the thirty-fourth vertebra (the end of the coccyx).

In the virtual spine model of the patient, based on his/her anthropometric dimensions, distances of individual planes from a base plane, which is the hip plane 18, were determined, from which at a distance x1 = 35 cm (distance between planes 18 and 17) the shoulder plane 17 is established and at a distance x2 = 62 cm (distance between planes 18 and 16) the head plane 16, at a distance x3 = 102 cm (distance between planes 18 and 20) the feet plane 20 and at a distance x4 = 54 cm (distance between planes 18 and 19) the knee plane 19. Sling hangers determined by planes 16, 17 and 18 are each equipped with two independent actuating elements. Sling hangers determined by planes 19 and 20 are each equipped with one actuating element.

Based on the diagnosis of the disease/degenerative condition and physiotherapeutic recommendations, the ranges of permissible displacements of the virtual cross-sectional planes installed at the suspension points were determined in the virtual environment.

The rehabilitation program was designed using the scaled virtual spine model of the patient in such a way that for the designed stage of rehabilitation, a physical therapy specialist set the virtual spine model in the desired rehabilitation position on a touch screen computer or in holographic glasses by moving all planes vertically. Additionally, he set the spine model by rotating the planes associated with the suspensions equipped with two independent actuating element around the suspension point and/or by tilting the virtual cross-sectional planes from the vertical. Moreover, he determined the implementation time of one setting, the speed of movement of the actuators, the number of repetitions and the length of the break after completing the exercises of a given stage of the rehabilitation process. After confirming the settings, repetitions and times, data regarding the displacements of the cross-sectional planes and the decreed time for maintaining the forced position as well as the number of repetitions of a given setting were transferred to a programming block of the rehabilitation process. Each of the subsequent stages of the rehabilitation process was designed in the same way.

In the programming block of the rehabilitation process 9, information about the change in the position of individual planes was transformed into signals controlling the operation of individual actuators, the movement of which forces the movement of virtual cross-sectional planes related to the assigned sling hangers. Signal sequences controlling the actuators for the implementation of the rehabilitation position for all stages of the rehabilitation process were stored as a rehabilitation program for the selected patient in a block constituting a database of rehabilitation programs.

Designing the rehabilitation program for the selected patient completed the preparatory stage for implementing the method for monitoring and visualizing the process of spine relieving rehabilitation according to the invention.

The implementation of the rehabilitation was initiated by downloading the rehabilitation program for the selected patient from the database of rehabilitation programs - 11. Running the program resulted in setting the actuators on axes of the support points at set distances x1 to x4 and setting the strings at the appropriate height above a rehabilitation bed. Then, the sling hangers were placed on the rehabilitation bed so that the suspension points coincided with the axes of the sling hangers.

The patient was placed on the sling hangers so that their axes ran through the suspension points around the head, shoulders, hips, knees and feet, and the sling hangers for head, shoulders and hips were each connected to 2 strings, while the sling hangers for knees and feet were connected to single strings.

After connecting all the sling hangers to the actuators, the patient was relieved by lowering the surface of the rehabilitation bed and the patient was suspended on sling hangers, the axes of which were located in the planes of the patient's suspension points, so that the strings of the actuators coincided with the virtual cross-sectional planes 16, 17, (18), 19 and 20. In this position, the patient was scanned and, in a visualization block 1, a hologram of the patient was generated using an image signal from scanning cameras 13, which moved, during scanning, at a constant speed over structural elements 15 of the device for spine rehabilitation 2. The hologram of the patient can also be prepared before suspending the patient. The hologram of the patient was archived in the database of virtual images of patients. Then, the rehabilitation program designed for the patient undergoing rehabilitation was implemented. The rehabilitation program was implemented by the movement of the actuators of the device for rehabilitation 2 based on the control program 9 previously prepared for the patient undergoing rehabilitation. In a spine movement visualization system 5, based on the signal sequences representing the direction and range of actuator movements performed for a given stage of the rehabilitation program, a new image of the spine model of the rehabilitated patient and an image of the location of the virtual cross-sectional planes 16, 17, 18, 19 and 20 connected to the virtual spine model were generated for each stage of rehabilitation. During the rehabilitation, the movement and change in the position of the virtual spine model of the patient were visualized in two directions vertical and horizontal and in rotation around the long axis of the spine, to the extent resulting from the applied stroke or movement of each actuator associated with a specific sling hanger and the cross-sectional plane associated with it. In the holographic glasses worn by the physical therapist supervising the rehabilitation process in an augmented virtual reality system the real image of the device for rehabilitation 2 from a first environment from the visualization block 1 was combined with the computer-generated hologram of the patient from a second environment from the database 3 and with the virtual image, correlated with the hologram, of his/her spine model from a third environment from the database 4. Throughout the entire period of implementing the rehabilitation program, the course of the rehabilitation procedure was monitored in real time by observing correlated images, the real image from the first environment and the patient's hologram generated in the visualization block 1, as well as the virtual image of the model of the rehabilitated patient's spine, with marked cross-sectional planes 16, 17, 18, 19 and 20. Based on the real image of the patient's movement on the device for rehabilitation 2 and the generated image of the spine model movement, in the holographic glasses to which the virtual image generated in the visualization block 1 was transmitted, the correctness of the scope of performing movements set in the program controlling 9 the device for rehabilitation 2 was verified by computer.

The created patient avatar along with the spine model and the suspension planes were modeled in the Unity 3D environment, which enabled their visualization using Hololens 12 glasses. Thanks to this procedure, the physical therapist supervising the rehabilitation process and equipped with Hololens glasses gained access to a mixed reality environment in which the real image of the surroundings along with the device for rehabilitation and the patient suspended in it, the patient's avatar and the virtual spine model along with the suspension planes that were superimposed on the semi-transparent patient's avatar, were present.

The device contained safety elements, namely a Safety circuit manually triggered by the patient in the event of pain and a safety button connected to the Safety circuit activated by the physical therapist supervising the rehabilitation process, which stopped the rehabilitation process. The supervisor also had the opportunity to correct the operating ranges of the actuators in individual positions in order to reduce the discomfort of the patient undergoing rehabilitation.

The device for relieving rehabilitation, protected by patents no. PL 226008 and US 2021/0100712 A1 owned by AGH, used in an exemplary embodiment of the method for monitoring and visualizing the process of spine relieving rehabilitation, was equipped with Siemens SIMATIC S7-1200F PLC controller, CPU 1215FC and SIMATIC KTP 700 7-inch HMI control panel. Electrically powered linear actuators Motec MD56-0-24-W6-456-756-110P002 were used as actuators. Each actuator had positioning feedback with a Hall sensor with a movement range of 50 to 400 mm. To scan a patient undergoing rehabilitation, ASC 10GO 1.67 SRM cameras were used, built on their own drive and moving on treadmills permanently mounted to the longer sides of a horizontal supporting frame 14 of the device for spine rehabilitation 2. The image of the spine with appropriate anthropometric parameters is superimposed on the obtained virtual image of the patient. In the planes of the suspensions, cross-sectional planes are generated in the virtual image, allowing to move them together with the virtual image of the patient and his/her spine. Each actuation of any actuators of the device causes a simultaneous displacement of the cross-sectional planes in the virtual image of the patient and his/her spine. The visualization process takes place in the holographic glasses, e.g. Hololens, and it is the so-called mixed reality.

The method of spine relieving rehabilitation and the device for spine relieving rehabilitation equipped with the system for implementing the method according to the invention will be used in all medical facilities dealing with spine rehabilitation. The possibilities offered by the system for rehabilitation according to the invention facilitate and accelerate the process of creating individualized, and therefore more effective, rehabilitation programs for specific patients. The method according to the invention enables real-time supervision of the safety and effectiveness of the rehabilitation program. The use of the invention will facilitate, accelerate and raise the spine rehabilitation process to a higher level.

### List of reference numerals

- 1 -: Visualization block - first environment,
- 2 -: Device for relieving rehabilitation,
- 3 -: Database of patients' holograms - second environment,
- 4 -: Database of spine models - third environment,
- 5 -: Visualization block of spine model movement,
- 6 -: Verification block,
- 7 -: Analysis block,
- 8 -: Database of tomographic examinations,
- 9 -: Central control unit - managing the device control program,
- 10 -: Cloud-server - library
- 11 -: Database of programs controlling the device for rehabilitation,
- 12 -: Holographic glasses
- 13 -: Scanning cameras,
- 14 -: Horizontal supporting frame of the device for rehabilitation,
- 15 -: Treadmill of the scanning camera,
- 16 -: Virtual cross-sectional plane at the head suspension point
- 17 -: Virtual cross-sectional plane at the shoulder suspension point,
- 18 -: Virtual cross-sectional plane at the hip suspension point,
- 19 -: Virtual cross-sectional plane at the knee suspension point,
- 20 -: Virtual cross-sectional plane at the feet suspension point,
- 21 -: Virtual correlated model of the patient's spine
- 22 -: Actuating elements implementing the relieving rehabilitation.

## Claims

1. A method for monitoring and visualizing the process of spine relieving rehabilitation, using a device for spine relieving rehabilitation and virtual and mixed reality technologies, as well as a virtual 3D model of the spine of a patient undergoing rehabilitation in this way, **characterized by** real-time monitoring a mixed reality image composed of a real image of the patient, a hologram of the patient and a virtual image of his/her spine model superimposed on it, as well as changes in the position thereof, generated by the rehabilitation process, which is the result of the following activities:
- downloading, from a database of typical virtual spine models, containing at least 6 different models, a virtual 3D spine model corresponding to the anthropometric data of the patient for whom the relieving rehabilitation process is being prepared,
- modifying a selected typical 3D spine model based on imaging diagnostics and the patient's anthropometric parameters, and introducing natural curvatures and all pathological changes shown in the imaging documentation, and scaling the model based on the patient's anthropometric dimensions and his holographic avatar, and archiving the 3D spine model adapted in this manner to the patient and his/her medical condition in a database of virtual images of patients' spines (3),
- downloading the adapted virtual 3D spine model of the patient and determining, based on his/her anthropometric dimensions, at least four and preferably five suspension points which determine the axes of sling hangers,
- generating virtual cross-sectional planes perpendicular to the long axis of the virtual spine model in the adapted virtual 3D spine model of the patient, at the determined suspension points,
- determining, for the rehabilitation program with five suspension points, a cross-sectional plane (16), at the shoulder suspension point, a cross-sectional plane (17), at the hip suspension point, a cross-sectional plane (18), at the knee suspension point, a cross-sectional plane (19), and at the feet suspension point, a cross-sectional plane (20),
- establishing, in the virtual spine model of the patient, on the basis of his/her anthropometric dimensions, distances of individual planes from a base plane, which is the hip plane (18), from which the shoulder plane (17) is established at a distance x1, head plane (16) is established at a distance x2, the feet plane (20) is established at a distance of x3, and the knee plane (19) is established at a distance of x4,
- determining a rehabilitation program using the scaled virtual spine model of the patient, displayed on a computer touch screen, on which individual virtual cross-sectional planes are moved vertically using holographic glasses and/or are rotated around the suspension point and/or are tilted from the vertical, thereby setting the virtual spine model in the desired rehabilitation position for a given stage of the rehabilitation process, and data regarding the displacements of the cross-sectional planes and the decreed time for maintaining the forced position are transferred to a programming block of the rehabilitation process (11), and the same procedure is followed when designing next stages of the rehabilitation process,
- transforming, in the programming block of the rehabilitation process, information about the change in the position of individual planes into signals by means of which the operation of individual actuating elements is controlled, causing the movement of virtual cross-sectional planes, thereby creating a sequence of signals controlling the actuating elements for implementing the rehabilitation position for each subsequent stage of the rehabilitation process, and storing signal sequences in a block constituting a database of rehabilitation programs,
- downloading the rehabilitation program dedicated to the patient and positioning the actuating elements (22), preferably linear actuators, each of which in pairs or individually is intended for one sling hanger, in the planes of the patient's suspension points, so that strings of the actuating elements coincide with the virtual cross-sectional planes (16), (17) and (18) and the strings of individual actuating elements coincide with the virtual cross-sectional planes (19) and (20),
- placing the patient on the sling hangers arranged at set distances x1 to x4, so that the suspension points coincide with the axes of the sling hangers,
- relieving the patient to the starting position or, optionally, before hanging, generating a hologram of the patient in a visualization block (1) using an image signal from scanning cameras (13), which move at a constant speed over structural elements (15) of a spine rehabilitation device (2), and archiving in the database of patients' virtual images (3),
- realizing the movement of the actuating elements of the device for rehabilitation (2) based on the program controlling (9) the device for rehabilitation (2) using the settings of selected movement planes set by a rehabilitation doctor/physiotherapist on the virtual image of the spine,
- transferring, to a spine movement visualization system (5), signals representing the amount and direction of movement of the actuating elements of the device for rehabilitation (2) that implement subsequent stages of the rehabilitation program,
- generating a new image of the spine model of the rehabilitated patient and an image of the position of virtual cross-sectional planes (16), (17), (18), (19) and (20) connected to the spine model, in the spine movement visualization system (5), based on the sequence of signals representing the direction and range of the actuating element movements carried out for a given stage of the rehabilitation program,
- visualizing, using the position of the virtual cross-sectional planes (16), (17), (18), (19) and (20) connected to the spine model, its movement in two directions vertical and horizontal and for the virtual cross-sectional planes (16), (17), (18) additionally in rotation around the long axis of the spine, to the extent resulting from the applied stroke or movement of each actuator from the pair of actuating elements (22) related to a specific sling hanger,
- in a mixed reality system, in the holographic glasses, combining the real image of the device for rehabilitation (2) from a first environment from the visualization block (1), with the computer-generated hologram of the patient from a second environment from the database (3) and with the virtual image, correlated with the hologram, of the model of his/her spine from a third environment from the database (4).
- real-time monitoring the course of the rehabilitation procedure by observing correlated images, the real image from the first environment and the patient's hologram generated in the visualization block (1), as well as the virtual image of the model of the rehabilitated patient's spine with marked cross-sectional planes (16), (17), (18), (19) and (20), which is superimposed on the semi-transparent hologram of the patient,
- based on the image of the patient's actual movement on the device for rehabilitation (2) and the image of the generated movement of the spine model, in the holographic glasses to which the virtual image generated in the visualization block (1) is transferred, verifying by computer the correctness of the range of movements set in the program controlling (9) the device for rehabilitation (2) and introducing necessary changes or restrictions for the settings specified in the unverified program.

2. The method for monitoring and visualizing the process of spine relieving rehabilitation according to claim 1, **characterized in that** the initial relieving of the patient, constituting the starting position for the rehabilitation process, is carried out by moving the strings of all sling hangers in the direction perpendicular to the plane of a bed by the same amount allowing to obtain space for executing all stages of the rehabilitation program or by lowering the bed by an amount allowing to obtain the required space, and the program is completed by setting a position analogous to the starting position and lowering the patient onto the bed or raising the bed to a position in which the patient's relieving position is eliminated.

3. The method for monitoring and visualizing the process of spine relieving rehabilitation according to claim 1, **characterized in that** the real image from the first environment (1), the patient's hologram from the second environment (3), and the virtual image of the spine model from the third environment (4) are stored in a cloud (10) (server) constituting the user's library,

4. The method for monitoring and visualizing the process of spine relieving rehabilitation according to claim 1, **characterized in that** the movement of the actuating elements (e.g. actuators) of the device for rehabilitation (2) is correlated with the movement of the virtual spine model (21), visualized on the virtual spine model, in third environment of the database (4), is limited by the permissible range of motion of the vertebrae and spine, used by medical specialists in clinical trials.

5. The system for monitoring and visualizing the process of spine relieving rehabilitation, using a device for spine relieving rehabilitation equipped with a set of scanning cameras and a virtual and mixed reality technology, as well as a virtual 3D model of the spine of a patient undergoing rehabilitation in this way, **characterized in that** to the longer sides of a horizontal supporting frame (14) of the device for spine relieving rehabilitation (2) treadmills are permanently mounted, each having one scanning camera (13) slidingly mounted therein.

6. The system for monitoring and visualizing the process of spine relieving rehabilitation according to claim 5, **characterized in that** inside the horizontal supporting frame (14) of the device for spine rehabilitation (2) five sliding beams are mounted, to which the actuating elements, preferably linear actuators, are slidably attached, along with strings and sling hangers attached to them, wherein the first beam is shifted relative to the central fixed beam by a distance of x1 set for the examined patient, the second beam by a distance of x2, the third beam by a distance of x3 and the fourth beam by a distance of x4, which are determined on the basis of anthropological parameters of the examined patient.

7. The system for monitoring and visualizing the process of spine relieving rehabilitation according to claim 5, **characterized in that** each actuating element is equipped with a sensor determining the amount and direction of the actual movement of a single string of each sling hanger, and each beam on which the actuating elements of the sling hangers are mounted is equipped with a sensor determining the amount of axial movement of the beam, with all movement signals for each element being transmitted to a spine movement visualization system.
